(19) Europäisches Patentamt
European Patent Office
Office européen des brevets

(11) **EP 3 193 802 B1**

(12) **EUROPEAN PATENT SPECIFICATION**

(45) Date of publication and mention
of the grant of the patent:
**20.11.2019 Bulletin 2019/47**

(21) Application number: **15770753.0**

(22) Date of filing: **15.09.2015**

(51) Int Cl.:
*A61F 13/476* *(2006.01)*   *A61F 13/56* *(2006.01)*
*A61F 13/515* *(2006.01)*   *A61F 13/512* *(2006.01)*
*A61F 13/15* *(2006.01)*   *A61F 13/514* *(2006.01)*

(86) International application number:
**PCT/US2015/050069**

(87) International publication number:
**WO 2016/044196 (24.03.2016 Gazette 2016/12)**

(54) **ADHESIVE STRIPING WITH APERTURED FILMS**

KLEBSTOFFSTREIFEN MIT PERFORIERTEN FOLIEN

BANDES ADHÉSIVES AVEC FILMS PERFORÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.09.2014 US 201462050328 P**

(43) Date of publication of application:
**26.07.2017 Bulletin 2017/30**

(73) Proprietor: **The Procter & Gamble Company**
**Cincinnati, OH 45202 (US)**

(72) Inventors:
• **TORO, Carlo**
**74564 Crailsheim (DE)**
• **CHEESEMAN, William, Winfield**
**Cincinnati, Ohio 45202 (US)**
• **OOTEN, David, Mark**
**Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**EP-A1- 0 978 263   WO-A1-91/18573**
**US-A- 5 722 966**

**Description**

FIELD OF THE INVENTION

Disclosed are absorbent articles comprising film laminates bonded with adhesive stripes.

BACKGROUND OF THE INVENTION

Absorbent articles comprise numerous components, typically layers of materials that are bonded together. The article components may be bonded through various means, but much of the bonding is done via adhesives. One difficulty of working with adhesives is the challenge of balancing the right application level of adhesive to achieve adequate bond strength with cost and with prevention of processing issues such as adhesive burn-through with thin films.

In simple terms, the more adhesive used, the stronger the adhesive bond. Though there may be many variables, the strength of a particular adhesive bond between two materials may be roughly proportional to the amount, or thickness of the adhesive used. To reduce the amount of the adhesive used may reduce the cost and reduce processing problems, but there is increased risk that the bond will not be adequately strong. Thus there is a continuing need for new adhesive applications and for tailoring adhesive use to particular materials.

SUMMARY OF THE INVENTION

An article having a longitudinal axis and a lateral axis, the article comprising a topsheet and a backsheet, wherein the topsheet comprises an apertured film and the backsheet comprises a film, wherein the topsheet film and the backsheet film are bonded via a plurality of adhesive stripes.

BRIEF DESCRIPTION OF THE FIGURES

Figure 1 shows a top plan view of the garment facing surface of a sanitary napkin according to the present invention.
Figure 2 is a transverse section of the sanitary napkin of Figure 1.

DETAILED DESCRIPTION OF THE INVENTION

Definitions

The term "absorbent article" is used herein to mean any article able to receive and/or absorb and/or contain and/or retain fluids and/or exudates, especially body fluids and exudates. "Absorbent articles" refer to, without being limited to, sanitary napkins, panty liners, incontinence pads, interlabial pads, baby diapers, adult incontinence diapers, and human waste management devices.

The term "disposable" is used herein to describe articles that are not intended to be laundered or otherwise restored or reused as an article (i.e., they are intended to be discarded after a single use.)

The term "topsheet" generally refers to the cover layer, in an absorbent article such as a diaper or catamenial pad, that faces the wearer of the absorbent article.

The term "longitudinal", as used herein, refers to a line, axis, or direction in the plane of the absorbent article that is generally aligned with (i.e., approximately parallel to) a vertical plane that bisects a standing wearer into left and right body halves when the absorbent article is worn.

The term "lateral", as used herein, refers to a line, axis, or direction that generally lies within the plane of the article that is generally perpendicular to the longitudinal direction.

The term "z-dimension" refers to the dimension orthogonal to the length and width of the layer, structure, or article. The z-dimension usually corresponds to the thickness of the layer, structure, or article.

In the present invention, adhesive striping may be used to bond film to film. While typical laminates are bonded with the adhesive applied across the entire surface area of the substrates being bonded, here the adhesive is applied is a striped pattern. And though each stripe may be thicker than the thickness of a planar application of adhesive, overall, there may be less adhesive used, in gsm, yet still have the result of greater bonding strength. The adhesive striping works especially well when bonding an uneven surface, such as a cone-shaped or apertured film. Without being bound by theory, it is thought that application of the adhesive in a striped pattern allows for the adhesive where applied to be thicker. That is, it allows for particular areas to have an abundance of adhesive. These thicker areas of adhesive are able to better penetrate and embed into the crevices of an uneven surface and consequently have stronger adhesion.

Having certain areas (the stripes) where there is thicker adhesive provides better mechanical adhesion and interlocking of the substrates being bonded, even though overall, less adhesive is used than if a planar layer of adhesive had been applied.

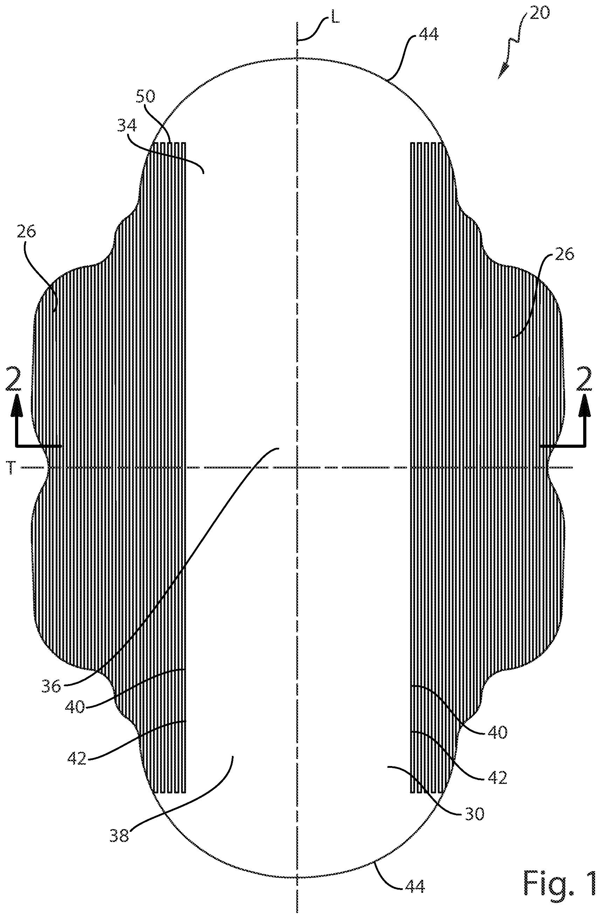

An absorbent article 20 for wearing in an undergarment is shown in FIG. 1. The absorbent article 20, and components thereof, may have a longitudinal direction, designated by the longitudinal centerline L, and a lateral direction, designated by the lateral centerline T. The longitudinal centerline may be oriented in the longest dimension of the absorbent article, and the lateral centerline may be orthogonal thereto. When a first location is nearer to the longitudinal centerline than a second location, the first location can be considered to be laterally inboard to the second location. Similarly, the second location can be considered laterally outboard from the first location. When a third location is nearer to the lateral centerline than a fourth location, the third location can be considered longitudinally inboard to the fourth location. Also, the fourth location can be considered longitudinally outboard from the third location. A reference to an inboard location, without a lateral or longitudinal limitation, refers to a location that is laterally inboard and/or longitudinally inboard to another location.

The absorbent article may comprise a backsheet, a topsheet, and an absorbent core positioned between the backsheet and the topsheet. The topsheet may comprise an apertured film. The topsheet and backsheet may each have a pair of wings or flaps 26.

The topsheet and the backsheet may each have a main body portion and may have a pair of wings 26 associated therewith. The main body portion 30 of the topsheet and/or backsheet may have two spaced apart longitudinal side edges 44 and two spaced apart lateral edges 42. The main body portion 30 may have a front region 34, a back region 38, and a central region 36 disposed between the front region and back region. The front region, central region, and back region are generally disposed along the longitudinal centerline of the article and generally divide the article into thirds along the longitudinal centerline.

Each of the wings is associated with the main body portion at a juncture 40. Each wing extends laterally outward from the longitudinal centerline and from each of the longitudinal side edges. The wings may extend laterally outward from each longitudinal side edge of the central region.

The topsheet and the backsheet may have approximately the same shape and size and be aligned with each other. The topsheet and backsheet may extend out from the longitudinal axis beyond the core, creating the wings. In such a case, the outline of the absorbent article will be the same whether viewed from the top or the bottom, such as in Fig. 1. An absorbent core may be positioned between the topsheet and the backsheet. The absorbent core may be a rectangular shape, and may be smaller than the topsheet and backsheet, in some cases not overlapping with the wing areas. Though in some cases, the core may overlap the wings.

As shown in Figure 1, the adhesive 50 is applied in the wing areas, 26. The adhesive may be applied to the backsheet film, which can then be bonded to the topsheet film (not shown). As shown in FIG. 1, the adhesive is applied in a plurality of longitudinally-oriented stripes on the wing areas.

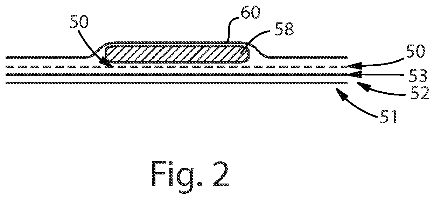

Figure 2 is a schematic of an article viewed towards the longitudinal axis along the line 2-2 in Fig. 1. The backsheet 52 forms the outer surface of the article. The backsheet may comprise a nonwoven 51 and a film 53, where the nonwoven forms the outermost surface, or if there is no backsheet nonwoven, the backsheet film may form the outermost surface. The adhesive 50 may be applied in stripes to the inner surface of the backsheet film 53. Along the sides, in the wing areas, the backsheet film 53 may be bonded via the adhesive stripes to the topsheet film 60. In the center of the article, adhesive stripes may bond the backsheet film 53 to the core 58.

The adhesive may be applied as continuous stripes from one end of the wing area in the front region to the opposite end in the back region. Each adhesive stripe may have a width, as measured along the lateral axis, of from about 0.5 mm to about 1.0 mm, or in some cases, from about 0.25, 0.3, 0.4, 0.5, 0.6, 0.7, 0.8, 0.9, 1.0, 1.1, 1.2, 1.3, 1.4, 1.5, 1.6, 1.7, 1.8, 1.9, or 2.0 mm to about 2.0, 1.9, 1.8, 1.7, 1.6, 1.5, 1.4, 1.3, 1.2, 1.1, 1.0, 0.9, 0.8, 0.7, 0.6, 0.5, 0.4, 0.3, or 0.25 mm, with every combination therein. Each adhesive stripe may have a thickness in the z-dimension from about 2 microns to about 100 microns, or in some cases from about 5, 10, 15, 20, 25, 30, 35, 40, 45, 50, 55, 60, 65, 70, or 75 microns to about 80, 75, 70, 65, 60, 55, 50, 45, 40, 35, 30, 25, 20, 15, 10, 5 or 2 microns, with every combination therein. The width of the space between adhesive stripes may be from about 0.25 mm to about 2.0 mm, or in some cases, 1.0 mm to about 1.5 mm. In some cases the width of the space between adhesive stripes may be from about 0.3, 0.4, 0.5, 0.6, 0.7, 0.8, 0.9, 1.0, 1.1, 1.2, 1.3, 1.4, 1.5, 1.6, 1.7, 1.8, 1.9, or 2.0 mm to about 2.0, 1.9, 1.8, 1.7, 1.6, 1.5, 1.4, 1.3, 1.2, 1.1, 1.0, 0.9, 0.8, 0.7, 0.6, 0.5, 0.4, 0.3, or 0.25 mm, with every combination therein. And the width of each adhesive stripe may be from about 25% to about 400% of the space between adjacent stripes, or in some cases, from about 30%, 40%, 50%, 60%, 70%, 75%, 80%, 90%, 100%, 110%, 120%, 130%, 140%, 150%, 160%, 170%, 180%, 190%, or 200% to about 200%, 190%, 180%, 170%, 160%, 150%, 140%, 130%, 120%, 110%, 100%, 90%, 80%, 70%, 60%, 50%, 40%, 30%, or 25%, with every combination therein, of the space between adjacent stripes. Similarly, the ratio of the of the width of the stripe to the width of the space between adjacent adhesive stripes may be from about 1:3 to about 3:1, or in some cases, the ratio may be from about 1:4 to about 4:1, or any ratio in between, in some cases about 1:4, 1.5:4, 1:2, 2.5:4, 3:4, 3.5:4, 1:1, 4:3.5, 4:3, 4:2.5, 2:1, 4:1.5, or 4:1.

Conventional adhesive bonding may be achieved by applying a continuous layer across the entire surface to be bonded. At typical levels for bonding a topsheet and a backsheet, for example about 13 gsm, the thickness of the adhesive layer in the z-dimension may be about 10-15 microns. In some cases, the adhesive applied via stripes, for example, in one wing, may be the same gsm and same thickness as when the adhesive is applied as a continuous layer. In such a case, the total amount of adhesive applied in the stripes wing will be less than the total amount of adhesive applied in the continuous layer wing, due to the space between the stripes. But the adhesive in the stripes wing may deliver equal bonding strength. Thus, the same bonding strength is achieved while saving on the amount of adhesive used.

In some cases, each adhesive stripe may be applied in a greater amount (greater thickness in the z-dimension) than the thickness of the adhesive that is applied over the entire surface. In such cases, the total amount of adhesive applied when using stripes may still be less than the total amount applied when covering the entire surface, yet the adhesive striping can still deliver superior bonding strength. In such a case, superior bonding strength is achieved while using less adhesive. The global basis weight of adhesive applied is at most equal to the local basis weight, for example, when the entire surface of a bonding area is covered with adhesive. When the adhesive is applied in stripes, for example, such that the entire surface of the bonding area is not covered, the global basis weight will be less than the local basis weight. Local basis weight may be defined as the basis weight for a particular stripe, and when referring to the basis weight of adhesive striping, it is understood that the basis weight is the local basis weight. In some embodiments, the global basis weight may be at most about 90% of the local basis weight. Global basis weight is calculated as the local basis weight times the ratio of the surface area covered by adhesive to the total area. In some embodiments, the global basis weight may be at most about 80%, 70%, 60%, 50%, 40%, 30%, or 20% of the local basis weight. In some embodiments, the global basis weight may be at most about 90% of the local basis weight, while the laminate exhibits adhesive strength of at least about 0.8 N/25mm, in some cases, about 0.7 N/25mm, 1.0 N/25mm, 1.5 N/25mm, or 2.0 N/25mm, as measured by the Standard Test Method for Peel Resistance of Adhesives (T-Peel Test) ASTM D-1876 with the modifications described below.

In some embodiments, an adhesive stripe may be continuously applied, or it may comprise a plurality of discrete elements. Whether the adhesive stripes are applied continuously or whether they comprise a plurality of discrete elements, the adhesive stripes may be arranged in a general linear or curvilinear formation. For example, at least one of the plurality of adhesive stripes may comprise a plurality of beads of adhesive that are generally arranged in the longitudinal direction. Moreover, one or more of the plurality of adhesive stripes may be continuous while the remaining number of the plurality of adhesive stripes is discontinuous.

The distance between adjacent adhesive stripes can vary within an area. For example, the distance, or width of the space between adhesive stripes in one portion of one area may be a certain amount, while the distance between adhesive stripes in a second portion of the area may be a different amount. Also, the adhesive stripes within an area, such as a wing, can have varying basis weights. For example, adhesive stripes in a portion of an area may have a local basis weight of 10 gsm while adhesive stripes in a second portion of the area may have a local basis weight of 15 gsm. In some cases, one of the films to be bonded by the adhesive stripes may be a topsheet film that is an apertured polymeric film web provided with a three-dimensional surface structure and/or with fluid transport apertures. Suitable topsheets and topsheet films may be such as those described in U.S. Ser. No. 12/193,325 or in U.S. Pat. Nos. 4,342,314, 4,609,518, 4,463,045, and 4,629,643. Apertured methods include, but are not limited to, the following: vacuum forming, hydroforming, needle punching (solid or hollow), hydrosonics, ultrasonics, and any combination thereof. In some embodiments, the film may comprise microtextures, i.e., microscopic patterns of surface aberrations to produce three dimensional surface structures. The three dimensional surface of the film may be comprised of microtextures or microapertures having a diameter of about 0.02 mm to about 0.2 mm, a height of about 0.02 mm to about 0.2 mm, and fluid transport apertures having a minimum diameter of about 0.05 mm. Topsheets may be made that comprise the apertured polymeric film web, again, such as described in U.S. Ser. No. 12/193,325. In some cases, an article may comprise a secondary topsheet that acts as an absorbent core as well. The secondary topsheet may be about the same width as the core, and may be placed between the topsheet and core. In this and other embodiments, the article may be considered to have a multi-layer core.

A backsheet typically may be a nonwoven bonded to a film, with the nonwoven providing the outside of the backsheet and of the article, while the film side faces inside the article. Suitable backsheets include those described in U.S. Ser. No. 11/087,475. The backsheet film may also have apertures.

The absorbent core, disposed between the topsheet and the backsheet, absorbs and retains bodily fluids that have penetrated the topsheet. The absorbent core may be any absorbent means which is capable of absorbing or retaining bodily liquids. Suitable materials and examples may be found in U.S. Ser. No. 11/087,475. The absorbent core may include any of a wide variety of liquid-absorbent materials commonly used in sanitary napkins and other absorbent articles, such as comminuted wood pulp, which is generally referred to as airfelt. Examples of other suitable absorbent materials for use in the absorbent core include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers;

absorbent gelling materials; or any equivalent material or combinations of materials, or mixtures of these, such as, for example, laminated structures comprising outer fibrous layers and particles of superabsorbent polymers comprised therebetween. The absorbent core may be manufactured in a wide variety of sizes and shapes (e.g., rectangular, oval, hourglass, dog bone, asymmetric, etc.).

In some embodiments, a plurality of adhesive stripes may be used to bond the absorbent core to the backsheet film. The adhesive may be applied in stripes along the entire backsheet film, in the wing areas and in the main body portion. In such a case, a bond is formed between the backsheet film and the topsheet film in the wing areas, and in the center of the article, the backsheet film is bonded to the core. In such a case, the article may comprise bonds via adhesive striping between a film and a film and also between a film and a nonwoven, such as described in U.S. filing 11/128,579. Additionally, any of the adhesive striping patterns and variations disclosed in U.S. 11/128,579 may also be appropriate to use in the present invention.

Optionally, the absorbent article may comprise all those components typical for the intended product use. For example, absorbent articles such as sanitary napkins and pantiliners can comprise components such as wings in order to improve their positioning and soiling protection performance, especially towards the rear end of the article. Such designs are shown, for example, in EP 130,848 or EP 134, 086, U.S. Design Patent 394,503. Wings can be provided as separate pieces and be attached to the article, typically a pantiliner or a sanitary napkin, or they can be integral with the materials of the absorbent articles, e.g., by being an integral extension of the topsheet, the backsheet, or a combination thereof.

In general, the direction of the adhesive stripes will be in the longitudinal direction of the absorbent article, or what is referred to as the machine-direction.

In some embodiments, in a set of parallel adhesive stripes, the stripes on the ends, for example, the stripes that are the most and least outboard from the centerline, may have a greater amount of adhesive than the other stripes in the set. In general, a wing area may have a first and second portion in which there is some variation, that is, in which, for example, the spacing of the stripes, the thickness or amount of the adhesive stripe, or the pattern of the stripes is different in the first portion from the second portion.

The adhesive stripes of the present invention may also provide benefits in addition to improved bonding strength. In some cases, the adhesive stripes may act as a barrier to fluid runoff from the topsheet. The adhesive stripes can create channels and through a capillary effect, the fluid may be redirected in a longitudinal direction rather than a lateral direction. This can then prevent an overflow of fluid in the lateral part of the wing area. In some cases, the striped adhesive in the wing areas may be colored so as to be visible through the topsheet. This may provide a visual cue to the user of an improved barrier to fluid. In some current articles, portions of the article are mechanically activated (ring-rolled), to help with the fit of the article in the user's clothing, but also as a visual signal to the user. The visual presence of the adhesive striping in the wing areas may be then be a substitute for the need to activate the article.

Examples

Table 1 shows data for four samples, a reference sample 1 and examples 1-3 wherein topsheet and backsheet laminates are bonded with a plurality of adhesive stripes. The laminates in all four samples was the same, that is, the materials bonded were a backsheet film bonded to an apertured topsheet film, for use, for example, as a wing laminate. The adhesive for all four samples was the same. The area of all four samples was the same, at 190 x 48, or 9,120 $mm^2$.

For the reference sample, the adhesive was applied as a continuous layer covering the entire area of the sample, at 13 gsm local basis weight. This resulted in 0.119 g of adhesive. The resulting bond strength, using the Standard Test Method for Peel Resistance of Adhesives, with modifications (as described below), resulted in an adhesive bond strength of 1.0 N/15mm.

For examples 1-3, 50% of the area was coated with adhesive stripes, using a 1-on-1-off slot adhesive pattern. The adhesive stripes had a lateral width of 1 mm and were spaced 1 mm apart. The adhesive in examples 1-3 was applied at a local basis weight of 13 gsm, 16 gsm, and 18 gsm, respectively. Each example thus used less adhesive than the reference sample. The table shows the resulting adhesive bond strengths, with example 3 being close to the same bond strength as the reference, though less adhesive was used.

Table 1: Topsheet to backsheet wing laminate

| | Adhesive application | Adhesive outer dimension Length x Width mm | Adhesive Outer dimension Area mm$^2$ | Surface area covered by adhesive mm$^2$ | Outer dimension covered by the adhesive % | Adhesive stripes width mm | space mm | Adhesive Global Basis Weight g/m2 | Adhesive Local Basis Weight | Adhesive g | Adhesive bond strength N/ 15mm |
|---|---|---|---|---|---|---|---|---|---|---|---|
| Ref 1 | Full Coating | 190x48 | 9120 | 9120 | 100% | - | - | 13 | 13 | 0.119 | 1.0 |
| Ex 1 | Plurality of stripes | 190x48 | 9120 | 4560 | 50% | 1.0 | 1.0 | 6.5 | 13 | 0.059 | 0.5 |
| Ex 2 | Plurality of stripes | 190x48 | 9120 | 4560 | 50% | 1.0 | 1.0 | 8.0 | 16 | 0.073 | 0.7 |
| Ex 3 | Plurality of stripes | 190x48 | 9120 | 4560 | 50% | 1.0 | 1.0 | 9.0 | 18 | 0.082 | 0.95 |

The relationship between global basis weight and local basis weight is the following:

$$\text{Global Basis Weight} = \text{Local basis weight} \times \text{Surface area covered by adhesive/Total Area}$$

Table2: Topsheet to backsheet wing laminate

| | | Adhesive outer dimension Length x Width mm | Adhesive Outer dimension Area mm$^2$ | Surface area covered by adhesive mm$^2$ | Outer dimension covered by the adhesive % | Adhesive stripes width mm | space mm | Adhesive Global Basis Weight t g/m2 | Adhesive Local Basis Weight | Adhesive g | Adhesive bond strength N/ 25mm |
|---|---|---|---|---|---|---|---|---|---|---|---|
| Ref 2 | Full Coating | 160x63 | 10080 | 10080 | 100% | - | - | 11 | 11 | 0.111 | 1.5 |
| Ex 4 | Plurality of stripes | 160x63 | 10080 | 6240 | 62% | 1.5 | 1.0 | 8.9 | 14.5 | 0.090 | 2.4 |
| Ex 5 | Plurality of stripes | 160x63 | 10080 | 6240 | 62% | 1.5 | 1.0 | 9.5 | 15.5 | 0.097 | 2.8 |
| Ex 6 | Plurality of stripes | 160x63 | 10080 | 6240 | 62% | 1.5 | 1.0 | 9.9 | 16.0 | 0.100 | 2.6 |

Example 2 shows data for four samples, a reference sample 2 and examples 4-6 wherein the backsheet film is bonded to the apertured topsheet film via a plurality of adhesive stripes. The laminates in all four samples was the same. In examples 4, 5, and 6, the adhesive stripes had a lateral width of 1.5 mm and were spaced 1 mm apart. The amount of the laminate covered by adhesive in the examples was 62%, (global basis weight is 62% of local basis weight), yet all three examples had a higher adhesive bond strength than the reference.

Table 3 shows data for four samples, a reference sample 3 and examples 7-9 wherein the backsheet film is bonded to the absorbent core via a plurality of adhesive stripes. The laminates in all four samples was the same.

Table 3: Core to backsheet laminate

| | Adhesive application | Adhesive outer dimension Length x Width mm | Adhesive Outer dimension Area mm² | Surface area covered by adhesive mm² | Outer dimension covered by the adhesive % | Adhesive stripes | space mm | Adhesive Global Basis Weight t g/m2 | Adhesive Local Basis Weight | Adhesive g | Adhesive bond strength N/50mm |
|---|---|---|---|---|---|---|---|---|---|---|---|
| Ref 3 | Full Coating | 284x55 | 15620 | 15620 | 100% | - | - | 5.0 | 5.0 | 0.0781 | 0.53 |
| Ex 7 | Plurality of stripes | 284x55 | 15620 | 7952 | 49.1% | 1 | 1 | 3.0 | 6.0 | 0.0477 1 | 0.77 |
| Ex 8 | Plurality of stripes | 284x55 | 15620 | 7952 | 49.1% | 1 | 1 | 3.6 | 7.0 | 0.0556 6 | 0.77 |
| Ex 9 | Plurality of stripes | 284x55 | 15620 | 7952 | 49.1% | 1 | 1 | 4.1 | 8.0 | 0.0636 2 | 0.94 |

Test Method

Standard Test Method for Peel Resistance of Adhesives (T-Peel Test) ASTM D-1876 with the following modification:

Cut the bonded panels into 25 mm wide test specimens. Other specimen widths may be used, provided the test machine grips are of ample width to apply the load uniformly across the width of the adherends. The 76-mm long unbonded ends bent apart, parallel to the glue line, for clamping in the grips of the testing machine. Clamp the bent, unbonded ends of the test specimen in the test grips of the tension testing machine. Apply the load at a constant head speed of 500 mm/min. Determine the peel resistance over the length of the bonded panel. Other unbonded ends length may be used, provided the ends do not slip inside the grips. The unbonded ends can be shorter to fit within the size of the article.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1.  An article having a longitudinal axis and a lateral axis, the article comprising a topsheet and a backsheet, the topsheet and backsheet each having a main body portion (30) and a pair of wings (26), wherein the topsheet comprises an apertured film and the backsheet comprises a film, wherein the topsheet film and the backsheet film are bonded via a plurality of longitudinally-oriented adhesive stripes on the wing areas.

2.  The article of claim 1, further comprising an absorbent core positioned between the topsheet and the backsheet.

3.  The article of claim 2, wherein the absorbent core and the backsheet film are bonded via a plurality of adhesive stripes.

4.  The article of claim 1, wherein the width of the space between adjacent adhesive stripes is at most about 2.0mm.

5.  The article of claim 1, wherein the width of the space between adjacent adhesive stripes is from about 0.25mm to about 2.0mm.

6.  The article of claim 1, wherein the width of the adhesive stripes is from about 0.25mm to about 2.0mm.

7.  The article of claim 1, wherein each stripe has a stripe width, and wherein the ratio of the width of the stripe to the width of the space between adjacent adhesive stripes is from about 1:3 to about 3:1.

8.  The article of claim 1, wherein each adhesive stripe has a thickness in the z-dimension from about 2 microns to about 100 microns.

9.  The article of claim 1, wherein the adhesive stripes comprise adhesive with a local basis weight from about 2 gsm to about 30 gsm.

10. The article of claim 1, wherein the article further comprises two wing areas wherein the topsheet and backsheet are directly joined together with no part of the core between the topsheet and the backsheet.

11. The article of claim 10, wherein the adhesive stripes cover less than about 85% of each wing area.

12. The article of claim 1, wherein the topsheet film is microtextured.

13. An article as claimed in any preceding claim, wherein the global basis weight is at most 90% of the local basis weight, with an adhesive bond strength of at least about .8 N/25mm as measured by the Standard Test Method for Peel Resistance of Adhesives (T-Peel Test) ASTM D-1876.

**Patentansprüche**

1.  Artikel mit einer Längsachse und einer Querachse, wobei der Artikel eine Oberschicht und eine Unterschicht umfasst, wobei die Oberschicht und die Unterschicht jeweils ein Grundelement (30) und ein Paar Flügel (26) aufweisen, wobei die Oberschicht eine Lochfolie umfasst und die Unterschicht eine Folie umfasst, wobei die Oberschichtfolie und die Unterschichtfolie über eine Vielzahl von in Längsrichtung ausgerichteter Klebstoffstreifen auf den Flügelflächen verbunden sind.

2. Artikel nach Anspruch 1, ferner umfassend einen Absorptionskern, der zwischen der Oberschicht und der Unterschicht angeordnet ist.

3. Artikel nach Anspruch 2, wobei der Absorptionskern und die Unterschichtfolie über eine Vielzahl von Klebstoffstreifen verbunden sind.

4. Artikel nach Anspruch 1, wobei die Breite des Zwischenraums zwischen benachbarten Klebstoffstreifen höchstens ungefähr 2,0 mm beträgt.

5. Artikel nach Anspruch 1, wobei die Breite des Abstands zwischen benachbarten Klebstoffstreifen von ungefähr 0,25 mm bis ungefähr 2,0 mm beträgt.

6. Artikel nach Anspruch 1, wobei die Breite der Klebstoffstreifen von ungefähr 0,25 mm bis ungefähr 2,0 mm beträgt.

7. Artikel nach Anspruch 1, wobei jeder Streifen eine Streifenbreite aufweist und wobei das Verhältnis der Breite des Streifens zur Breite des Abstands zwischen benachbarten Klebstoffstreifen von ungefähr 1:3 bis ungefähr 3:1 beträgt.

8. Artikel nach Anspruch 1, wobei jeder Klebstoffstreifen eine Dicke in der z-Dimension von ungefähr 2 Mikrometer bis ungefähr 100 Mikrometer aufweist.

9. Artikel nach Anspruch 1, wobei die Klebstoffstreifen Klebstoff mit einem lokalen Basisgewicht von ungefähr 2 Gramm pro Quadratmeter bis ungefähr 30 Gramm pro Quadratmeter umfassen.

10. Artikel nach Anspruch 1, wobei der Artikel ferner zwei Flügelflächen umfasst, wobei die Oberschicht und die Unterschicht direkt miteinander verbunden sind, wobei kein Teil des Kerns zwischen der Oberschicht und der Unterschicht ist.

11. Artikel nach Anspruch 10, wobei die Klebstoffstreifen weniger als ungefähr 85 % jeder Flügelfläche bedecken.

12. Artikel nach Anspruch 1, wobei die Oberschichtfolie mikrotexturiert ist.

13. Artikel nach einem der vorstehenden Ansprüche, wobei das globale Basisgewicht höchstens 90 % des lokalen Basisgewichts beträgt, mit einer Klebebindungsstärke von mindestens ungefähr 0,8 N/25 mm, wie durch das Standardprüfverfahren für Schälwiderstand von Klebstoffen (T-Schältest) ASTM D-1876 gemessen.


## Revendications

1. Article ayant un axe longitudinal et un axe latéral, l'article comprenant une feuille de dessus et une feuille de fond, la feuille de dessus et la feuille de fond ayant chacune une partie de corps principale (30) et une paire d'ailettes (26), dans lequel la feuille de dessus comprend un film ouvert et la feuille de fond comprend un film, dans lequel le film de feuille de dessus et le film de feuille de fond sont liés par l'intermédiaire d'une pluralité de bandes adhésives orientées longitudinalement sur les zones d'ailette.

2. Article selon la revendication 1, comprenant en outre une âme absorbante positionnée entre la feuille de dessus et la feuille de fond.

3. Article selon la revendication 2, dans lequel l'âme absorbante et le film de feuille de fond sont liés par l'intermédiaire d'une pluralité de bandes adhésives.

4. Article selon la revendication 1, dans lequel la largeur de l'espace entre des bandes adhésives adjacentes est un maximum d'environ 2,0 mm.

5. Article selon la revendication 1, dans lequel la largeur de l'espace entre des bandes adhésives adjacentes va d'environ 0,25 mm à environ 2,0 mm.

6. Procédé selon la revendication 1, dans lequel la largeur des bandes adhésives va d'environ 0,25 mm à environ 2,0 mm.

7. Article selon la revendication 1, dans lequel chaque bande a une largeur de bande, et dans lequel le rapport de la largeur de la bande à la largeur de l'espace entre des bandes adhésives adjacentes va d'environ 1:3 à environ 3:1.

8. Article selon la revendication 1, dans lequel chaque bande adhésive a une épaisseur dans la dimension z allant d'environ 2 microns à environ 100 microns.

9. Article selon la revendication 1, dans lequel les bandes adhésives comprennent un adhésif avec un poids de base local allant d'environ 2 g/m$^2$ à environ 30 g/m$^2$.

10. Article selon la revendication 1, l'article comprenant en outre deux zones d'ailette dans lesquelles la feuille de dessus et la feuille de fond sont directement reliées l'une à l'autre sans aucune partie de l'âme entre la feuille de dessus et la feuille de fond.

11. Article selon la revendication 10, dans lequel les bandes adhésives recouvrent moins d'environ 85 % de chaque zone d'ailette.

12. Article selon la revendication 1, dans lequel le film de feuille de dessus a une microtexture.

13. Article tel que revendiqué dans une quelconque revendication précédente, dans lequel le poids de base global est un maximum de 90 % du poids de base local, avec une résistance de liaison adhésive d'au moins environ 0,8 N/25 mm telle que mesurée par le procédé de test standard pour la résistance au pelage des adhésifs (test de pelage T) ASTM D-1876.

Fig. 1

50 60 58 50 53 52 51

Fig. 2

**REFERENCES CITED IN THE DESCRIPTION**

**Patent documents cited in the description**

- US 193325
- US 4342314 A
- US 4609518 A
- US 4463045 A
- US 4629643 A
- US 087475
- US 128579
- EP 130848 A
- EP 134086 A
- US 394503 A